**Europäisches Patentamt**

**European Patent Office**

**Office européen des brevets**

(11) Veröffentlichungsnummer: **0 453 589 B1**

## EUROPÄISCHE PATENTSCHRIFT

(45) Veröffentlichungstag der Patentschrift: **28.12.94**

(51) Int. Cl.5: **A61N 1/36**, A61N 1/08

(21) Anmeldenummer: **90107693.5**

(22) Anmeldetag: **24.04.90**

(54) **Anordnung zur Gewebestimulation.**

(43) Veröffentlichungstag der Anmeldung:
**30.10.91 Patentblatt 91/44**

(45) Bekanntmachung des Hinweises auf die
Patenterteilung:
**28.12.94 Patentblatt 94/52**

(84) Benannte Vertragsstaaten:
**DE FR GB IT NL SE**

(56) Entgegenhaltungen:
**EP-A- 0 334 681**
**DE-A- 2 254 928**
**US-A- 4 402 322**

(73) Patentinhaber: **Siemens Elema AB**
**Röntgenvägen 2**
**S-171 95 Solna 1 (SE)**

(84) Benannte Vertragsstaaten:
**SE**

(73) Patentinhaber: **SIEMENS AKTIENGESELL-**
**SCHAFT**
**Wittelsbacherplatz 2**
**D-80333 München (DE)**

(84) Benannte Vertragsstaaten:
**DE FR GB IT NL**

(72) Erfinder: **Andersson, Peter, Dipl.-Ing**
**Högalidsgatan 38A**
**S-11730 Stockholm (SE)**
Erfinder: **Bengtson, Bo, Dipl.-Ing**
**Karlslundsgatan 20**
**S-15137 Södertälje (SE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Anordnung zur Gewebestimulation bei einem Lebewesen mit einem Stimulationsimpulsgenerator zur Erzeugung von Stimulationsimpulsen, mit einer Detektoreinrichtung zur Erfassung der Reaktion des Gewebes auf die Stimulation und mit einer den Stimulationsgenerator steuernden Steuereinrichtung, die zur Ermittlung der Stimulationsempfindlichkeit des Gewebes eine Verringerung der Energie der Stimulationsimpulse ausgehend von einem über der Stimulationsempfindlichkeit liegenden Wert veranlaßt, bis eine Reaktion des Gewebes ausbleibt.

Bei einer derartigen aus der DE-AS-22 54 928 bekannten Anordnung handelt es sich um einen Herzschrittmacher, mit dem das Herz eines Patienten mit Hilfe von Stimulationsimpulsen angeregt wird. Dabei erfolgt eine Kontraktion als Reaktion des Herzens nur dann, wenn die Energie der Stimulationsimpulse eine bestimmte, von Patient zu Patient variierende Stimulationsempfindlichkeit oder Reizschwelle des Herzgewebes übersteigt. Um den Energieverbrauch des bekannten Herzschrittmachers gering zu halten, wird anstelle einer Stimulation mit einer ständig über der aktuellen Stimulationsempfindlichkeit liegenden Energie die Energie jeweils aufeinanderfolgender Stimulationsimpulse schrittweise verringert und dabei die Reaktion oder Antwort des Herzens auf die Stimulation überwacht. Bleibt eine solche Reaktion aus, so wird die Stimulationsenergie auf einen mit Sicherheit über der Stimulationsempfindlichkeit des Herzens liegenden Wert erhöht und derselbe Vorgang wiederholt. Dadurch wird eine Anpassung der Stimulationsenergie und damit des Energieverbrauchs innerhalb des Herzschrittmachers an die veränderbare Stimulationsempfindlichkeit des Herzens angepaßt.

Bei einer ähnlichen aus der EP-OS 0 236 562 bekannten Anordnung zur Herzstimulation wird nach der Detektion einer auf eine Stimulation folgenden Reaktion die Stimulationsenergie jedesmal dann um einen bestimmten Wert verringert, wenn auch bei den zwei unmittelbar vorangegangenen Stimulationsereignissen eine Herzreaktion detektiert wurde. Wird dagegen auf die aktuelle Stimulation keine Herzreaktion registriert, so wird das Herz mit einem Stimulationsimpuls mit maximaler Energie stimuliert und für den nächsten Stimulationsimpuls ein gegenüber dem erfolglosen Stimulationsimpuls um einen bestimmten Wert erhöhter Energieinhalt festgesetzt.

Bei der aus EP-A-0334 681 bekannten Anordnung erfolgt eine Anpassung der Stimulationsenergie entweder in festen Zeitabständen oder auf Befehl eines externen Programmierers.

Der Erfindung liegt die Aufgabe zugrunde, eine Anordnung zur Gewebestimulation anzugeben, bei der einerseits die abgegebene Stimulationsenergie an den sich verändernden Verlauf der Stimulationsempfindlichkeit des Gewebes angepaßt ist, andererseits aber der Patient nicht ständig durch ausbleibende Stimulationen als Folge des Unterschreitens der Stimulationsempfindlichkeit belastet wird.

Gemäß der Erfindung wird die Aufgabe dadurch gelöst, daß bei der Anordnung der eingangs angegebenen Art die Steuereinrichtung die Ermittlung der Stimulationsempfindlichkeit in periodischen Zeitabständen durchführt und daß der Zeitabstand zwischen einer aktuellen Ermittlung der Stimulationsempfindlichkeit und der nächsten Ermittlung in Abhängigkeit von der Differenz zwischen dem aktuell ermittelten Wert der Stimulationsempfindlichkeit und dem zuvor ermittelten Wert der Stimulationsempfindlichkeit bemessen wird, wobei der Zeitabstand mit zunehmender Differenz verringert und mit abnehmender Differenz vergrößert wird.

Der wesentliche Vorteil der erfindungsgemäßen Anordnung besteht darin, daß die Ermittlung der Stimulationsempfindlichkeit nicht ständig, sondern in bestimmten Zeitabständen erfolgt, deren Länge in Abhängigkeit von der Änderung der Stimulationsempfindlichkeit variiert, so daß bei nur geringen Änderungen der Stimulationsempfindlichkeit diese in größeren Zeitabständen bei entsprechend geringer Belastung des Patienten neu ermittelt wird, während bei größeren Änderungen der Stimulationsempfindlichkeit der Zeitabstand zwischen aufeinanderfolgenden Ermittlungen der Stimulationsempfindlichkeit verringert wird, um deren Änderungen folgen zu können. Die Belastung des Patienten durch erfolglose Stimulationsversuche, wie sie bei jedem einzelnen Ermittlungsvorgang auftreten, wird auf diese Weise maßgeblich verringert.

Vorzugsweise wird in der Steuereinrichtung die zur Bemessung des Zeitabstandes herangezogene Differenz zwischen dem aktuell und dem zuvor ermittelten Wert der Stimulationsempfindlichkeit auf den Zeitabstand zwischen diesen beiden Ermittlungsvorgängen normiert. Dadurch wird der Zeitabstand zwischen jeweils aufeinanderfolgenden Ermittlungsvorgängen der Stimulationsempfindlichkeit an die Änderung der Stimulationsempfindlichkeit des zu stimulierenden Gewebes pro Zeiteinheit, also an die Änderungsgeschwindigkeit angepaßt.

Um zwischen den einzelnen Ermittlungsvorgängen eine sichere Stimulation des Gewebes ohne erfolglose Stimulationsversuche sicherzustellen, ist im Rahmen der Erfindung vorgesehen, daß der Stimulationsimpulsgenerator nach jeder Ermittlung der Stimulationsempfindlichkeit bis zu deren nächstfolgenden Ermittlung Stimulationsimpulse abgibt, deren Energie dem aktuell ermittelten Wert der Stimulationsempfindlichkeit zuzüglich einem Sicherheitswert entspricht. Dieser Sicherheitswert ist dabei in Bezug auf die jeweilige Zeitdauer bis zur nächsten Ermittlung der Stimulationsempfindlichkeit so bemessen, daß bei einem Ansteigen der Stimulationsempfindlichkeit während dieser Zeitdauer die Energie der erzeugten Stimulationsimpulse jederzeit über der Stimulationsempfindlichkeit des Gewebes liegt.

Entsprechend einer vorteilhaften Weiterbildung der erfindungsgemäßen Anordnung ist vorgesehen, daß der Stimulationsimpulsgenerator für die Ermittlung der Stimulationsempfindlichkeit biphasische Stimulationsimpulse und danach bis zur nächsten Ermittlung der Stimulationsempfindlichkeit monophasische Stimulationsimpulse erzeugt. Mit biphasischen Stimulationsimpulsen, wie sie z.B. aus der DE-OS 23 42 030 oder aus der DE-OS 26 19 001 bekannt sind, wird nämlich erreicht, daß die durch den Stimulationsimpuls im Gewebe erzeugten Polarisationserscheinungen so rasch verschwinden, daß eine sichere Erfassung der Reaktion des Gewebes auf die Stimulation möglich ist. Allerdings ist der Energiebedarf derartiger biphasischer Impulse im Vergleich zu monophasischen Impulsen relativ groß. Da jedoch von der erfindungsgemäßen Anordnung die biphasischen Stimulationsimpulse nicht ständig, sondern nur in bestimmten Zeitabständen zur Ermittlung der Stimulationsempfindlichkeit des Gewebes erzeugt werden, während das Gewebe in der Zwischenzeit mit monophasischen Stimulationsimpulsen erregt wird, ist der zur Stimulation erforderliche Energiebedarf insgesamt nur gering.

Die biphasischen Stimulationsimpulse weisen vorzugsweise jeweils zwei energiegleiche Teilimpulse unterschiedlicher Polarität mit einer zwischen ihnen liegenden Pause auf.

Zur Erläuterung der Erfindung wird im Folgenden auf die Figuren der Zeichnungen Bezug genommen; im einzelnen zeigen:

FIG 1 ein Blockschaltbild eines Ausführungsbeispiels der erfindungsgemäßen Anordnung,

FIG 2 in einem Zeitdiagramm unterschiedliche Stimulationsimpulse und zu ihrer Erzeugung erforderliche Steuersignale,

FIG 3 ein Beispiel für den zeitlichen Verlauf der Stimulationsempfindlichkeit eines zu stimulierenden Gewebes und den Verlauf der daran angepaßten Stimulationsenergie und

FIG 4 ein Flußdiagramm mit einzelnen, die Funktion der Anordnung in FIG 1 erläuternden Verfahrensschritten.

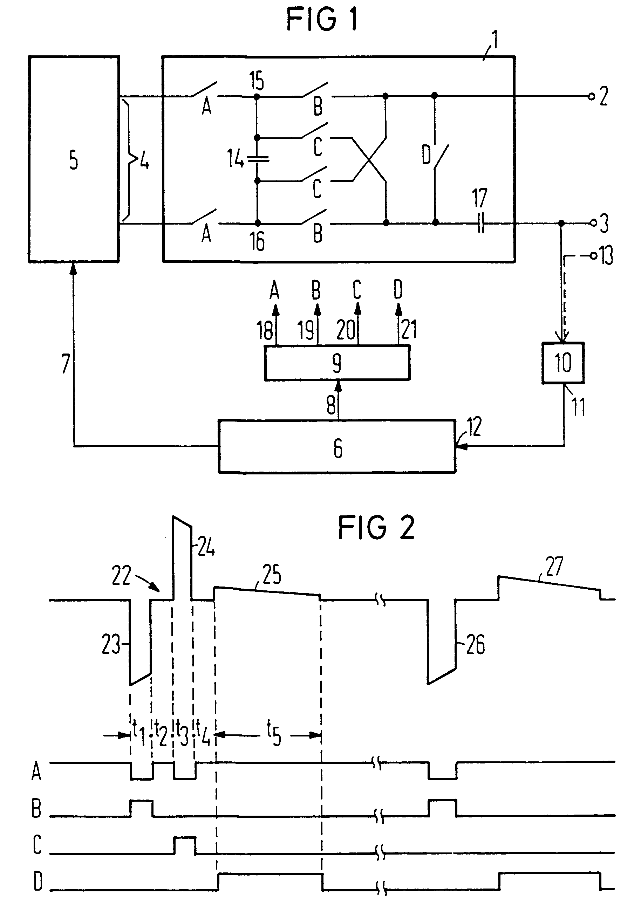

FIG 1 zeigt als Ausführungsbeispiel für die erfindungsgemäße Anordnung das Blockschaltbild eines Herzschrittmachers zur Gewebestimulation, in diesem Fall der Stimulation eines Herzens. Der Herzschrittmacher enthält einen Stimulationsimpulsgenerator (1), an dem ausgangsseitig eine indifferente Elektrode (2) und eine Stimulationselektrode (3) angeschlossen ist. Die indifferente Elektrode (2) wird von dem hier nicht dargestellten Gehäuse des Herzschrittmachers gebildet, während die Stimulationselektrode (3) in dem Herzen plaziert ist. Der Stimulationsimpulsgenerator (1) ist eingangsseitig an dem Ausgang (4) eines Stimulationsspannungsgenerators (5) angeschlossen, der den Stimulationsimpulsgenerator (1) mit einer vorgebbaren Spannung für die zu erzeugenden Stimulationsimpulse versorgt. Der jeweilige Wert für diese Spannung wird durch eine Steuereinrichtung (6) vorgegeben, die über eine Steuerleitung (7) mit dem Stimulationsspannungsgenerator (5) verbunden ist. Die Steuereinrichtung (6) ist ferner über eine weitere ausgangsseitige Steuerleitung (8) mit einer Zeitgebereinrichtung (9) verbunden, die, wie nachstehend noch erläutert wird, den zeitlichen Verlauf der Erzeugung der Stimulationsimpulse in dem Stimulationsimpulsgenerator (1) steuert.

Die Stimulationselektrode (3) ist mit einer Detektoreinrichtung (10) verbunden, die nach einem Stimulationsimpuls die Reaktion des stimulierten Gewebes erfaßt; an ihrem Ausgang (11) ist die Detektoreinrichtung (10) mit einem Steuereingang (12) der Steuereinrichtung (6) verbunden. Die Detektoreinrichtung (10) kann anstelle mit der Stimulationselektrode (3) auch mit einer in deren Nähe angeordneten Meßelektrode (13) verbunden sein, wie dies in FIG 1 gestrichelt dargestellt ist.

Innerhalb des Stimulationsimpulsgenerators (1) ist ein Speicherkondensator (14) an seinen Anschlüssen (15,16) über ein erstes Schalterpaar A mit dem Ausgang (4) des Stimulationsspannungsgenerators (5) verbunden. Der Speicherkondensator (14) ist ferner über ein zweites Schalterpaar B und ein drittes Schalterpaar C, die in einer Brückenschaltung angeordnet sind, mit der indifferenten Elektrode (2) und über einen Ausgangskondensator (17) mit der Stimulationselektrode (3) verbunden. Je nachdem, ob das Schalterpaar B oder das Schalterpaar C schließt, wird der Speicherkondensator (14) an seinem mit (15) bezeichneten Anschluß mit der indifferenten Elektrode (2) und an seinem Anschluß (16) mit dem Ausgangskondensator (17) verbunden oder umgekehrt mit seinem Anschluß (16) an der indifferenten Elektrode (2) und mit dem Anschluß (15) an dem Ausgangskondensator (17) angeschlossen. Schließlich ist ein Schalter D vorgesehen, der die Stimulationselektrode (3) über den Ausgangskondensator (17) mit der indifferenten Elektrode (2) verbindet. Die Schalterpaare A, B und C und der Schalter D werden über ihnen zugeordnete Steuerleitungen (18,19,20,21) von der Zeitgebereinrichtung (9) einzeln angesteuert, wie dies im folgenden anhand von FIG 2 erläutert wird.

FIG 2 zeigt in einem Zeitdiagramm ein Beispiel für einen biphasischen Stimulationsimpuls (22), der aus einem negativen Teilimpuls (23) gefolgt von einem positiven Teilimpuls (24) und einem Schnellentladeimpuls (25) besteht; ferner sind ein monophasischer Stimulationsimpuls (26) sowie die zur Erzeugung der Stimulationsimpulse (22,26) von der Zeitgebereinrichtung (9) an die Schalterpaare A,B und C sowie den Schalter D abgegebenen, hier entsprechend bezeichneten Steuersignale zu sehen. Dabei bedeutet ein von Null abweichender Signalzustand, daß der zugeordnete Schalter geschlossen ist.

Solange das Schalterpaar A geschlossen ist, wird der Speicherkondensator (14) auf die von der Steuereinrichtung (6) vorgegebene und von dem Stimulationsspannungsgenerator (5) erzeugte Ausgangsspannung aufgeladen. Zur Erzeugung des biphasischen Stimulationsimpulses zwischen der indifferenten Elektrode (2) und der Stimulationselektrode (3) wird zunächst das Schalterpaar B für eine Zeitdauer $t_1$ und nach einer anschließenden Pause $t_2$ das Schalterpaar C für eine Zeitdauer $t_3$ geschlossen; schließlich wird nach einer weiteren Pause $t_4$ der Schalter D für eine Zeitdauer $t_5$ geschlossen, wodurch der Ausgangskondensator (17) schnellentladen wird. Mit dem so erzeugten biphasischen Stimulationssignal (22) werden die aufgrund der Stimulation im Bereich der Stimulationselektrode (3) im Gewebe erzeugten Polarisationserscheinungen so schnell abgebaut, daß eine sichere, d.h. von Überlagerungen durch die Polarisationserscheinungen weitestgehend freie Erfassung der Reaktion des Gewebes auf die biphasische Stimulation mittels der Detektoreinrichtung (10) möglich ist. Wird die Reaktion des stimulierten Gewebes über die der Stimulationselektrode (3) benachbarte Meßelektrode (13) erfaßt, so kann anstelle des biphasischen Stimulationsimpulses (22) der einfacher zu erzeugende monophasische Stimulationsimpuls (26) zur Gewebestimulation verwendet werden; dies liegt daran, daß das Aktionspotential im Gewebe mit einer Zeitverzögerung von der Stimulationselektrode (2) kommend zur Meßelektrode (13) gelangt, so daß in dieser Zeit die Polarisationserscheinungen weitestgehend abgeklungen sind.

Der mit (26) bezeichnete monophasische Stimulationsimpuls wird durch Schließen des Schalterpaares B erzeugt. Anschließend wird der Ausgangskondensator (17) durch Schließen des Schalters D mit einem Schnellentladeimpuls (27) entladen. Wie FIG 2 zeigt, ist der Energieinhalt des monophasischen Stimulationsimpulses (26) bei gleicher Amplitude etwa um die Hälfte geringer als der des biphasischen Stimulationsimpulses (22). Beide Stimulationsimpulse (22, 26) zeigen jedoch trotz ihres unterschiedlichen Energieinhalts bei der Gewebestimulation etwa die gleiche Wirkung. Dies liegt daran, daß die Stimulationsempfindlichkeit des Gewebes nicht nur von dem tatsächlichen Energieinhalt des jeweiligen Stimulationsimpulses sondern auch von seiner Form abhängt. Wenn daher im folgenden von Stimulationsenergie in Bezug auf die Stimulationsempfindlichkeit des Gewebes die Rede ist, so sind zwei unterschiedliche Stimulationsimpulse dann als energiegleich anzusehen, wenn sie die gleiche Wirkung bei der Gewebestimulation zeigen.

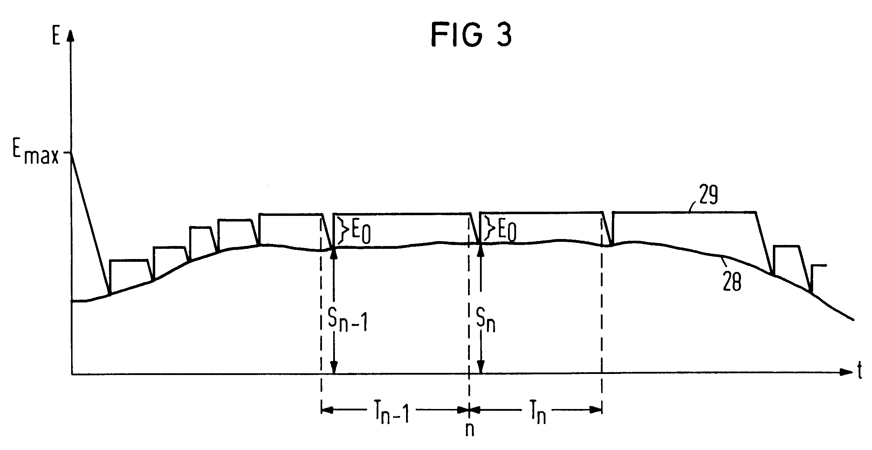

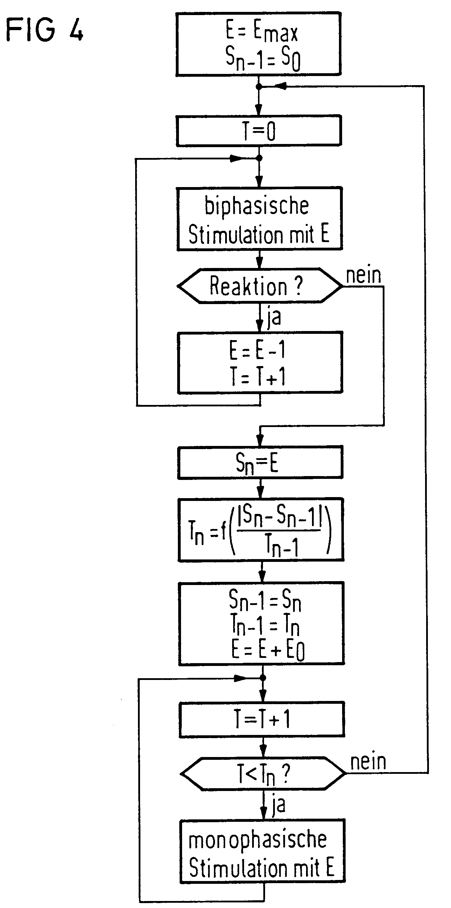

In FIG 3 ist mit (28) ein möglicher Verlauf der Stimulationsempfindlichkeit des zu stimulierenden Gewebes in Abhängigkeit von der Zeit t dargestellt. Die Stimulationsempfindlichkeit wird durch einen Energiewert E ausgedrückt, den ein Stimulationsimpuls mindestens für eine erfolgreiche Stimulation des Gewebes aufweisen muß. Um jederzeit eine erfolgreiche Stimulation sicherzustellen, könnte das Gewebe ständig mit einer maximalen Energie $E_{max}$ stimuliert werden. Der Energiebedarf wäre in diesem Fall jedoch sehr hoch. Daher wird, wie im folgenden erläutert wird, die Stimulationsenergie entsprechend dem Verlauf der mit (29) bezeichneten Kurve und den in FIG 4 in Form eines Flußdiagramms dargestellten Verfahrensschritten an die veränderliche Stimulationsempfindlichkeit des Gewebes angepaßt.

Zu einem in FIG 3 mit n bezeichneten Zeitpunkt wird die Stimulationsempfindlichkeit des Gewebes ermittelt. Für die Stimulationsempfindlichkeit wurde zuletzt vor einem Zeitabstand $T_{n-1}$ ein Wert $S_{n-1}$ ermittelt; anschließend wurde das Gewebe bis zu dem Zeitpunkt n mit einer um einen Sicherheitswert $E_0$ über der ermittelten Stimulationsempfindlichkeit $S_{n-1}$ liegenden Stimulationsenergie (Kurve 29) erregt. Zur erneuten Ermittlung der Stimulationsempfindlichkeit wird ein Zeitmesser T (FIG 4) auf den Wert Null zurückgestellt. Daraufhin wird das Gewebe mit dem biphasischen Stimulationsimpuls (22) (FIG 2) und der zuletzt verwendeten Stimulationsenergie $E = S_{n-1} + E_0$ beaufschlagt und unmittelbar darauf eine mögliche Reaktion des Gewebes mittels der Detektoreinrichtung (10) erfaßt. Wird eine Reaktion des stimulierten Gewebes detektiert, so wird die Stimulationsenergie E um eine Energiestufe verringert, der Zeitmesser T inkrementiert und nach einem hier nicht bezeichneten Stimulationszeitintervall ein erneuter biphasischer Stimulationsimpuls (22) dieses Mal mit der verringerten Stimulationsenergie E erzeugt. Dieser Vorgang wird solange wiederholt, bis eine Reaktion des Gewebes ausbleibt, weil die aktuelle Stimulationsempfindlichkeit $S_n$ unterschritten wurde. Die Energieänderung bei dem biphasischen Stimulationsimpuls (22) kann durch Variation der Impulsdauer und/oder Impulsamplitude erfolgen. In Abhängigkeit von der Differenz zwischen der soeben detektierten aktuellen Stimulationsempfindlichkeit $S_n$ und der davor zuletzt ermittelten Stimulationsempfindlichkeit $S_{n-1}$ bezogen auf den Zeitabstand $T_{n-1}$ zwischen den beiden Ermittlungsvorgängen für die Stimulationsempfindlichkeit wird ein neuer Zeitabstand $T_n$ vom Beginn der aktuellen Ermittlung bis zu der nächsten Ermittlung der Stimulationsempfindlichkeit festgelegt. Anschließend wird das Gewebe mit den monophasischen Stimulationsimpulsen (26) (FIG 2) und einer um den Sicherheitswert $E_0$ über der ermittelten Stimulationsempfindlichkeit $S_n$ liegenden Stimulationsenergie $E = S_n + E_0$ erregt, bis der nach jeder neuen Stimulation inkrementierte Zeitmesser T den Ablauf der Zeitdauer $T_n$ anzeigt. Anschließend wird die Stimulationsempfindlichkeit des Gewebes wie oben beschrieben erneut ermittelt.

Während der monophasischen Stimulation kann periodisch, beispielsweise nach jedem zehnten Stimulationsimpuls, ein biphasischer Stimulationsimpuls (22) erzeugt werden und mittels der Detektoreinrichtung (10) überprüft werden, ob die Stimulationsenergie zur Gewebestimulation noch ausreichend ist. Ist dies nicht der Fall, so wird die Stimulationsenergie vergrößert. Wenn die Detektoreinrichtung (10) an der Meßelektrode (13) angeschlossen ist, kann bei monophasischer Stimulation die Überprüfung auf ausreichende Stimulationsenergie laufend nach jedem Stimulationsimpuls (26) erfolgen.

Mittels der erfindungsgemäßen Anordnung wird die Stimulationsenergie (29) an den Verlauf der Stimulationsempfindlichkeit (28) angepaßt, wobei jedoch die Belastung des Patienten durch ausbleibende Stimulation so gering wie möglich ist.

Bezugszeichenliste

| | |
|---|---|
| 1 | Stimulationsimpulsgenerator |
| 2 | indifferente Elektrode |
| 3 | Stimulationselektrode |
| 4 | Ausgang von 5 |
| 5 | Stimulationsspannungsgenerator |
| 6 | Steuereinrichtung |
| 7 | Steuerleitung |
| 8 | weitere Steuerleitung |
| 9 | Zeitgebereinrichtung |
| 10 | Detektoreinrichtung |
| 11 | Ausgang von 10 |
| 12 | Steuereingang von 6 |
| 13 | Meßelektrode |
| 14 | Speicherkondensator |
| 15,16 | Anschlüsse von 14 |
| 17 | Ausgangskondensator |
| 18,19,20,21 | Steuerleitungen für A,B,C,D |
| 22 | biphasischer Stimulationsimpuls |
| 23 | negativer Teilimpuls |
| 24 | positiver Teilimpuls |
| 25 | Schnellentladeimpuls |
| 26 | monophasischer Stimulationsimpuls |
| 27 | Schnellentladeimpuls |
| 28 | Verlauf der Stimulationsempfindlichkeit |
| 29 | Verlauf der Stimulationsenergie |
| A | erstes Schalterpaar |
| B | zweites Schalterpaar |
| C | drittes Schalterpaar |
| D | Schalter |
| E | Energie |
| $E_{max}$ | maximale Energie |
| $E_0$ | Sicherheitswert |
| n | Zeitpunkt |
| $S_n$ | aktuell ermittelte Stimulationsempfindlichkeit |
| $S_{n-1}$ | zuletzt ermittelte Stimulationsempfindlichkeit |
| $S_0$ | Anfangswert für $S_{n-1}$ |
| t | Zeit |
| T | Zeitmesser |
| $T_n$ | Zeitabstand |
| $T_{n-1}$ | Zeitabstand |
| $t_1$ | Zeitdauer von 23 |
| $t_2$ | Pause zwischen 23 und 24 |
| $t_3$ | Zeitdauer von 24 |
| $t_4$ | Pause zwischen 24 und 25 |
| $t_5$ | Zeitdauer von 25 |

**Patentansprüche**

1. Anordnung zur Gewebestimulation bei einem Lebewesen mit einem Stimulationsimpulsgenerator (1) zur Erzeugung von Stimulationsimpulsen (22,26), mit einer Detektoreinrich tung (10) zur Erfassung der Reaktion des Gewebes auf die Stimulation und mit einer den Stimulationsimpulsgenerator (1) steuernden Steuereinrichtung (6), die zur Ermittlung der Stimulationsempfindlichkeit des Gewebes eine Verringerung der Energie der Stimulationsimpulse (22,26) ausgehend von einem über der Stimulationsempfindlichkeit liegenden Wert veranlaßt, bis eine Reaktion des Gewebes ausbleibt, **dadurch gekennzeichnet,** daß die Steuereinrichtung (6) die Ermittlung der Stimulationsempfindlichkeit in periodischen Zeitabständen $T_{n-1}$, $T_n$ durchführt und daß der Zeitabstand $T_n$ zwischen einer aktuellen Ermittlung der Stimulationsempfindlichkeit $S_n$ und der nächsten Ermittlung in Abhängigkeit von der Differenz zwischen dem aktuell ermittelten Wert der Stimulationsempfindlichkeit $S_n$ und dem zuvor ermittelten Wert der Stimulationsempfindlichkeit $S_{n-1}$ bemessen wird, wobei der Zeitabstand $T_n$ mit zunehmender Differenz verringert und mit abnehmender Differenz vergrößert wird.

2. Anordnung nach Anspruch 1, **dadurch gekennzeichnet,** daß in der Steuereinrichtung (6) die zur Bemessung des Zeitabstandes $T_n$ herangezogene Differenz zwischen dem aktuell und dem zuvor ermittelten Wert der Stimulationsempfindlichkeit $S_n$ bzw. $S_{n-1}$ auf den Zeitabstand $T_{n-1}$ zwischen diesen beiden Ermittlungsvorgängen normiert wird.

3. Anordnung nach Anspruch 1 oder 2, **dadurch gekennzeichnet,** daß der Stimulationsimpulsgenerator (1) nach jeder Ermittlung der Stimulationsempfindlichkeit $S_n$ bis zu deren nächstfolgenden Ermittlung Stimulationsimpulse (26) abgibt, deren Energie dem aktuell ermittelten Wert der Stimulationsempfindlichkeit $S_n$ zuzüglich einem Sicherheitwert $E_0$ entspricht.

4. Anordnung nach einem der vorangehenden Ansprüche, **dadurch gekennzeichnet,** daß der Stimulationsimpulsgenerator (1) für die Ermittlung der Stimulationsempfindlichkeit biphasische Stimulationsimpulse (22) erzeugt und danach bis zur nächsten Ermittlung der Stimulationsempfindlichkeit monophasische Stimulationsimpulse (26) erzeugt.

5. Anordnung nach Anspruch 4, **dadurch gekennzeichnet,** daß die biphasischen Stimulationsimpulse (22) jeweils zwei energiegleiche Teilimpulse (23,24) unterschiedlicher Polarität mit einer zwischen ihnen liegenden Pause $t_2$ aufweisen.

**Claims**

1. Device for tissue stimulation in a living organism, having a stimulation pulse generator (1) for generating stimulation pulses (22, 26), having a detector device (10) to detect the reaction of the tissue to the stimulation and having a control device (6), which controls the stimulation pulse generator (1) and which, to determine the stimulation sensitivity of the tissue, causes a reduction of the energy of the stimulation pulses (22, 26), starting from a value lying above the stimulation sensitivity, until a reaction of the tissue is absent, characterized in that the control device (6) carries out the determination of the stimulation sensitivity at periodic time intervals $T_{n-1}$, $T_n$, and in that the time interval $t_n$ between a current determination of the stimulation sensitivity $S_n$ and the next determination is dimensioned as a function of the difference between the currently determined value of the stimulation sensitivity

$S_n$ and the previously determined value of the stimulation sensitivity $S_{n-1}$, the time interval $T_n$ being reduced as the difference increases and being increased as the difference decreases.

2. Device according to Claim 1, characterized in that in the control device (6) the difference, utilized for dimensioning the time interval $T_n$, between the currently and the previously determined value of the stimulation sensitivity $S_n$ and $S_{n-1}$ respectively is normalized with respect to the time interval $T_{n-1}$ between these two determination procedures.

3. Device according to Claim 1 or 2, characterized in that after each determination of the stimulation sensitivity $S_n$ until the next following determination thereof, the stimulation pulse generator (1) emits stimulation pulses (26), the energy of which corresponds to the currently determined value of the stimulation sensitivity $S_n$ plus a safety margin $E_0$.

4. Device according to one of the preceding Claims, characterized in that for the determination of the stimulation sensitivity the stimulation pulse generator (1) generates biphasic stimulation pulses (22) and thereafter, until the next determination of the stimulation sensitivity, generates monophasic stimulation pulses (26).

5. Device according to Claim 4, characterized in that the biphasic stimulation pulses (22) exhibit in each instance two equal-energy partial pulses (23, 24) of differing polarity with a pause $t_2$ lying between them.

**Revendications**

1. Dispositif pour stimuler des tissus chez un être vivant, comportant un générateur d'impulsions de stimulation (1) servant à produire des impulsions de stimulation (22,26), un dispositif de détection (10) pour détecter la réaction du tissu à la stimulation et un dispositif de commande (6), qui commande le générateur d'impulsions de stimulation (1) et qui, pour la détermination de la sensibilité du tissu à la stimulation, déclenche une réduction de l'énergie des impulsions de stimulation (22,26) à partir d'une valeur supérieure à la sensibilité à la stimulation, jusqu'à ce que le tissu ne réagisse plus, caractérisé par le fait que le dispositif de commande (6) exécute la détermination de la sensibilité à la stimulation pendant des intervalles de temps périodiques $T_{n-1}$, $T_n$, et que l'intervalle de temps $T_n$ entre une détermination actuelle de la sensibilité à la stimulation $S_n$ et

la détermination suivante est mesuré en fonction de la différence entre la valeur actuelle déterminée de la sensibilité à la stimulation $S_n$ et la valeur déterminée auparavant de la sensibilité à la stimulation $S_{n-1}$, l'intervalle de temps $T_n$ étant réduit lorsque la différence augmente et étant accru lorsque la différence diminue.

2. Dispositif suivant la revendication 1, caractérisé par le fait que dans le dispositif de commande (6), la différence, utilisée pour le dimensionnement de l'intervalle de temps $T_n$, entre la valeur actuelle déterminée et la valeur déterminée auparavant de la sensibilité à la stimulation, à savoir $S_n$ et $S_{n-1}$, est normalisée en étant rapportée à l'intervalle de temps $T_{n-1}$ entre ces deux processus de détermination.

3. Dispositif suivant la revendication 1 ou 2, caractérisé par le fait qu'après chaque détermination de la sensibilité à la stimulation $S_n$, le générateur d'impulsions de stimulation (1) délivre, jusqu'à la détermination immédiatement suivante de cette sensibilité, des impulsions de stimulation (26), dont l'énergie correspond à la valeur, actuelle déterminée, de la sensibilité à la stimulation $S_n$, plus une valeur de sécurité $E_0$.

4. Dispositif suivant l'une des revendications précédentes, caractérisé par le fait que le générateur d'impulsions de stimulation (1) produit, pour la détermination de la sensibilité à la stimulation, des impulsions de stimulation biphasées (22) et ensuite, jusqu'à la détermination suivante de la sensibilité à la stimulation, délivre des impulsions de stimulation monophasées (26).

5. Dispositif suivant la revendication 4, caractérisé par le fait que les impulsions de stimulation biphasées (22) comprennent respectivement deux impulsions partielles (23,24) possédant une même énergie et des polarités différentes, avec une pause $t_2$ intercalée entre ces impulsions partielles.

## FIG 1

## FIG 2

FIG 3

FIG 4

$E = E_{max}$
$S_{n-1} = S_0$

$T = 0$

biphasische
Stimulation mit E

Reaktion ?  — nein

ja

$E = E - 1$
$T = T + 1$

$S_n = E$

$T_n = f\left(\dfrac{|S_n - S_{n-1}|}{T_{n-1}}\right)$

$S_{n-1} = S_n$
$T_{n-1} = T_n$
$E = E + E_0$

$T = T + 1$

$T < T_n$ ?  — nein

ja

monophasische
Stimulation mit E